(19) Europäisches Patentamt
European Patent Office
Office européen des brevets

(11) Publication number: **0 547 681 A2**

# (12) EUROPEAN PATENT APPLICATION

(21) Application number: **92203846.8**

(22) Date of filing: **10.12.92**

(51) Int. Cl.[5]: **C07K 7/06**, C07K 7/08, C07K 7/10, A61K 39/21

(30) Priority: **18.12.91 US 811047**

(43) Date of publication of application: **23.06.93 Bulletin 93/25**

(84) Designated Contracting States: **CH DE FR GB IT LI NL**

(71) Applicant: **MERCK & CO. INC.**
**126, East Lincoln Avenue P.O. Box 2000**
**Rahway New Jersey 07065-0900(US)**

(72) Inventor: **Tolman, Richard L.**
**29 Upper Warren Way**
**Warren, NJ 07059(US)**
Inventor: **Hannah, John**
**155 Idlebrook Lane**
**Matawan, NJ 07747(US)**

(74) Representative: **Barrett-Major, Julie Diane et al**
**Merck & Co., Inc. European Patent Department Terlings Park Eastwick Road**
**Harlow Essex CM20 2OR (GB)**

## (54) Synthetic peptides comprising a cyclic HIV principal neutralizing determinant and a lipopeptide.

(57) A synthetic peptide comprising a cyclic HIV principal neutralizing determinant and a microbial outer membrane lipopeptide analog may be used to raise high titers of HIV neutralizing antibodies in a mammal, in the absence of added adjuvant.

EP 0 547 681 A2

BACKGROUND OF THE INVENTION

Immunogens directed at production of immune responses against human immunodeficiency virus (HIV) are under active development worldwide. Acquired immune deficiency syndrome (AIDS) and other diseases associated with HIV infection are reaching epidemic proportions on a global basis. This invention was made in response to the need for effective immunogens which are able to induce immune responses directed against the etiologic agent of these diseases.

Use of complete viral proteins, such as gp120 or gp160 has met with limited success as a vaccine candidate, as the subunit proteins appear to induce a number of immunological responses, not all of which are beneficial in preventing HIV infection.

Presentation of peptidyl epitopes displayed by the intact virus or its subunit proteins, has also been attempted. Thus, peptide epitopes have been conjugated to carrier proteins or they have been produced as fusion proteins with carrier proteins.

Of particular interest in this regard are HIV peptidyl epitopes derived from the V3 domain of gp120, referred to hereinafter as principal neutralizing determinant (PND) peptides. These are peptide epitopes against which HIV neutralizing antibodies are directed. A preferred subset of the PND sequences are those comprising the sequence Gly Pro Gly or Gly Pro Gly Arg [Seq. ID:1:]. This sequence is found in a great many isolates of HIV in the third variable region of the HIV envelope protein gp120, and appears to be part of an important epitope, proper exposure of which to mammalian immune systems results in induction of HIV neutralizing immune responses.

The above noted approaches hold promise, but the immunogens are complicated to produce, and successful HIV vaccines are not available. Those in clinical study are not conjugate vaccines and do not provide reproducibly high levels of neutralizing antibodies. Experimental conjugate vaccines are difficult to prepare and parameters of loading, sterility, and pyrogenicity are difficult to control. Replacement of the carrier protein with a low molecular weight moiety with known structure and composition to form a hybrid molecule will avoid these problems. Hybrid peptides are easier to prepare and are not subject to the same regulatory requirements as carrier protein conjugates.

A desirable method of producing an effective immunogen would be a completely synthetic route which yelds a low molecular weight compound which is immunogenic. It is also desirable that the synthetic immunogen be capable of priming both B-cell and T-cell mediated immune responses, because HIV is known to multiply by cell-cell fusion (syncytia formation) and also to be present in the bloodstream where antibodies may bind to and neutralize the virus.

T-cell mitogens of various types are known, but immunologically effective hybrid peptides comprising cyclic HIV $V_3$-loop sequences have not been reported, nor are hybrid peptides known which comprise a small cyclic HIV principal neutralizing determinant and an epitope related to the N-terminus of the E. coli outer membrane lipopeptide.

Researchers working with the lipopeptide of the E. coli outer membrane have, over the years, synthesized and characterized low molecular weight analogs thereof. Thus, Wiesmuller et al., [Hoppe-Seyler's Z. Physiol. Chem., 364, 593-606 (1983)] described and synthesized the N-terminal pentapeptide of the E. coli lipoprotein by coupling S-[2,3-bis(palmitoyloxy)-(SRS)-propyl]-N-palmitoyl-R-cysteine to O-tert-butyl-seryl-o-tert-butyl-seryl-asparaginyl-alanine tert-butyl ester, followed by deprotection with trifluoroacetic acid.

Jung et al., [Liebigs. Ann. Chem., 1608-1622 (1983)] confirmed the mitogenic activity of E. coli lipoprotein N-terminal synthetic analogs in a mouse spleen cell model. Bessler et al., [J. Immunol. 135, 1900-1905 (1985)] compared the ability of S-(2,3-bis-(palmitoyloxy)-(2-R,S)-propyl)-N-palmitoyl-(R)-cysteine, -cysteine methyl ester, -cysteinyl-serine, -cysteinyl-seryl-serine, -cysteinyl-seryl-seryl-asparagine, and -cysteinyl-seryl-seryl-asparaginyl-alanine analogs for mitogenic activity, concluding that analogs having 2-5 amino acids exhibit strong stimulatory activity toward B-lymphocytes.

Jung et al., [Angew. Chem. Int. Ed. Engl. 24, 872-873 (1985)] reported on the increased production of epidermal growth factor receptor (EGF-R) specific antibodies upon coupling of a linear EGF-R peptide to N-palmitoyl-S-[(2RS)-2,3-bis(palmitoyloxy)propyl]cysteinyl serine (hereinafter referred to as $(Pam)_3$ Cys-Ser).

Reitermann et al., [Biol. Chem. Hoppe-Seyler 370, 343-352 (1989)] reported on additional analogs of $Pam_3$ Cys-Ser, including $(Pam)_3$Cys-Ser-$Lys_4$, $(Pam)_3$Cys-Ala-Gly, and noted the ability of these analogs to boost immune responses against the hapten dinitrophenol.

Deres et al., [Nature 342, 561-564 (1989)] reported that a linear synthetic influenza peptide covalently linked to $(Pam)_3$Cys-Ser-Ser could efficiently prime an influenza-virus-specific cytotoxic lymphocyte response in mice, in the absence of any other adjuvant.

Likewise, Wiesmuller et al., [Vaccine 7, 29-33 (1989)] reported on the ability of $(Pam)_3$ Cys Ser Ser covalently linked to a linear foot and mouth disease virus (FMDV) sequence to raise FMDV specific antibodies in guinea pigs.

Loleit et al., [Biol. Chem. Hoppe-Seyler, 371, 967-975 (1990)] reported on conjugates of $Pam_3$ Cys Ser Ser and linear peptides from three different isolates of human immunodeficiency virus. A similar disclosure is to be found in Jung et al., EP 0431 327 A1. Thus, the use of $(Pam)_3$ Cys Ser Ser conjugates has been noted in the art, but $(Pam)_3$ Cys Ser Ser hybrids with cyclic peptides have not been reported.

As a further enhancement to the mitogenic activity of the lipopeptide, Kurimura et al., [Chem. Pharm. Bull. 38(4), 1110-1112 (1990) and Peptide Chemistry 1990: Y. Shimonishi (Ed.), Protein Research Foundation, Osaka, 37-42 (1991)] noted that tripalmitoyl cysteinyl-seryl-seryl-asparaginyl-alanine derivatives are more mitogenic when the lipopeptide has the natural 2R stereochemistry, rather than 2S or a racemic stereochemistry at this position. Furthermore, these workers have noted a further enhancement of lipopeptide induced mitogenic activity when the palmitoyl residue on the cysteine of the lipopeptide is replaced with trichloroethoxy carbonyl (Troc). Additionally, Tsuda et al [Peptide Chemistry 1990: Y. Shimonishi (Ed.), Protein Research Foundation, Osaka, 131-134 (1991)] have shown that a tripalmitoyl lipopeptide, derived from Streptomyces willmorei, having a cysteinyl-asparaginyl-seryl glycyl-glycyl-serine structure, is active as a stimulator of bone marrow cells.

These findings are applied herein to provide an optimally active lipopeptide-HIV cyclic PND immunogen.

## SUMMARY OF THE INVENTION

A synthetic hybrid immunogen comprising a cyclic human immunodeficiency virus (HIV) principal neutralizing determinant (PND) coupled with a lymphocyte stimulatory epitope which is an analog of the E. coli or S. willmorei lipopeptide N-terminus, is useful to induce B and T cell immune responses against the epitope expressed by the cyclic portion of the immunogen, and is useful in the treatment or prevention of HIV infection.

## DETAILED DESCRIPTION OF THE INVENTION

A synthetic hybrid-immunogen comprising a triacyl-glycerol cysteinyl seryl serine or cysteinyl-asparaginyl-seryl-glycyl-glycyl-serine moiety linked to a cyclic HIV principal neutralizing determinant peptide may be prepared by separately preparing each subportion of the hybrid, and then linking the two subportions. A tripalmitoyl-glycerol linked cysteine $(Pam)_3$Cys-OH moiety may be prepared according to methods disclosed in the literature. Thus, the methods of Wiesmuller et al [Hopper-Seyler's Z Physiol Chem 364, 593-606 (1983)], Jung et al [Liebigs Ann. Chem. 1608-1622 (1983)], Bessler et al, [J. Immunol 135, 1900-1905 (1985)], Jung et al [Angew. Chem. Int. Ed. Engl 24, 872-873 (1985)], and Jung, EP 0431 327 AI may be used to prepare $(Pam)_3$ Cys-OH. This material is also commercially available from Boehringer Mannheim, Mannheim, FRG. For the synthesis of 2R palmitoyl and Troc contaning lipopeptide, the procedure of Kurimura et al [Peptide Chemistry 1990: Y. Shimonishi (Ed) Protein Research Foundation, Osaka, p 37-42 and 131-134 (1991)] is followed.

According to the instant invention, a linear peptide bearing an HIV PND is prepared by standard methods of solid phase peptide synthesis (see Solid Phase Peptide Synthesis, 1984, by Stewart and Young). The linear peptide is then acylated by the $(Pam)_3$Cys-OH at the peptide's free amino terminus, care having been taken to ensure that all other free amino groups in the peptide are appropriately protected.

Following acylation, the protecting groups are cleaved from the peptide, which is then cyclized. One simple method of cyclization is to incorporate two cysteines into the primary sequence of the linear peptide and then to allow oxidation of the peptide to form the cyclic disulfide. Other methods of cyclization, however, are equally applicable, such as thioether bond or amide bond formation of a cycle.

Thus, in a preferred embodiment of this invention, the hybrid immunogen has the general structure [Seq. Id:19:]:

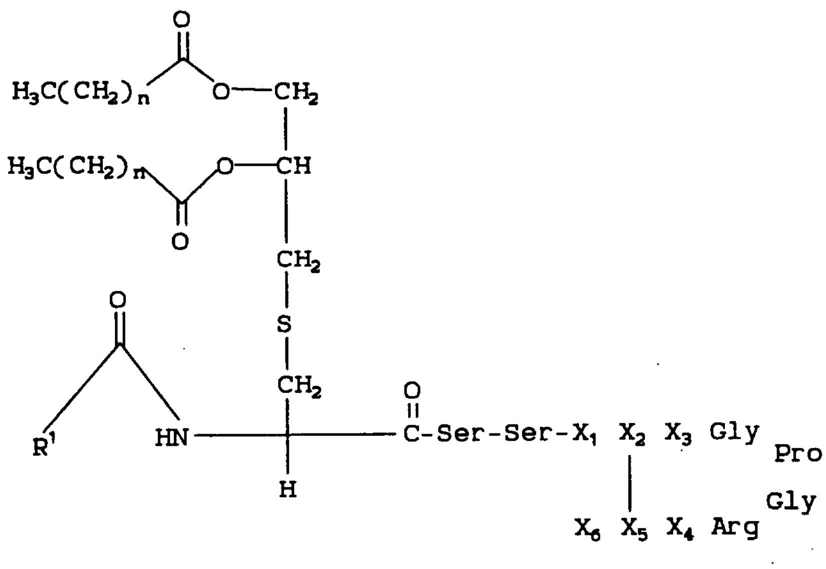

wherein:

- $X_1$ is a bond, an amino acid, or a peptide of 2-5 amino acids;
- $X_2$ is an amino acid bonded to $X_5$;
- $X_3$ is 1 to 25 amino acids long, selected from the sequence of amino acids found in any of the common isolates of HIV gp120, amino terminal and adjacent to the Gly Pro Gly Arg [Seq. Id:1:] sequence;
- $X_4$ is a bond or 1 to 25 amino acids long, selected from the sequence of amino acids found in any of the common isolates of HIV gp120, carboxy-terminal and adjacent to the [Seq. Id:1:] Gly Pro Gly Arg sequence;
- $X_5$ is an amino acid bonded to $X_2$;
- $X_5$ is hydroxyl, $-NH_2$, or an amino acid or a peptide of between 2 and 10 amino acids long; n is between 1 and 20, and is preferably 14; and $R^1$ is $CH_3(CH_2)_n$-or $Cl_3CCH_2O$-. Furthermore, the preferred stereochemistry is 2R.

This immunogen may be further defined according to the HIV cPND wherein $X_1$ is Nle, $X_2$ and $X_5$ are cysteine, $X_3$ $X_4$, and $X_5$ are as defined above, and n = 14 such that the hybrid immunogen has the formula [Seq. Id:20:]:

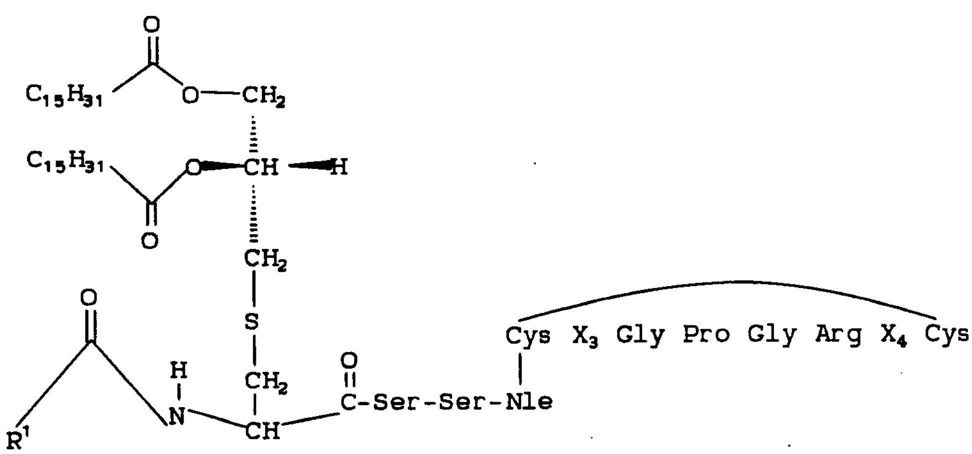

Guidance as to the sequences which are preferred for $X_3$ and $X_4$ is provided by LaRosa et al, Science 249, 932-935 (1990), wherein the sequence of 245 HIV isolates was examined, any of which sequences surrounding the Gly Pro Gly Arg may be used herein.

A consensus sequence for $X_3$ may be defined from that work as:
Thr Arg Pro Asn Asn Asn Thr Arg Lys Ser Ile His Ile [Seq. Id:15:]
and for $X_4$ as:
Tyr Thr Thr Gly Glu Ile Ile Gly Asp Ile Arg Gln Ala His [Seq. Id:16:]

Preferred sequences for $X_3$ and $X_4$ are those wherein:

$X_3$ is selected from:
Ile, His Ile, Ile His Ile, Arg Ile His Ile [Seq. Id:2:],
Lys Arg Ile His Ile [Seq. Id:3:], Arg Lys Arg Ile His Ile [Seq. Id:4:],
Lys Arg Lys Arg Ile His Ile [Seq. Id:5:], Asn Lys Arg Lys Arg Ile His Ile [Seq. Id:6:], Tyr Asn Lys Arg Lys Arg Ile His Ile [Seq. Id:7:],
Arg, and Gln Arg; and

$X_4$ is selected from:
Ala
Ala Phe, Ala Phe Tyr, Ala Phe Tyr Thr [Seq. Id:8:],
Ala Phe Tyr Thr Thr [Seq. Id:9:], Ala Phe Tyr Thr Thr Lys [Seq. Id:10:],
Ala Phe Tyr Thr Thr Lys Asn [Seq. Id:11:], Ala Phe Tyr Thr Thr-Lys Asn Ile [Seq. Id:12:], Ala Phe Tyr Thr Thr Lys Asn Ile Ile [Seq. Id:13:],
and Ala Phe Tyr Thr Thr Lys Asn Ile Ile Gly [Seq. Id:14:].

Within the scope of this invention, the compound cPND [Seq.Id:17:]:

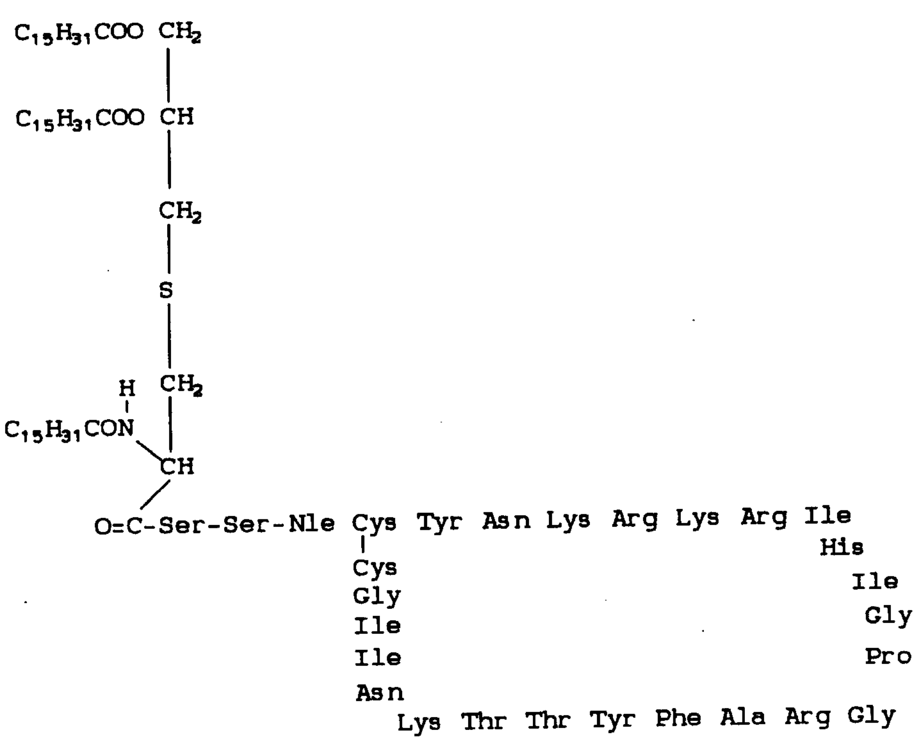

was prepared.

The lipopeptide - HIV cPND hybrid of this invention is not readily aqueous soluble. Thus, emulsions of this material may be prepared prior to administration, by Dounce homogenization, sonication or any other effective means. A dose of between about 1$\mu$g to 1 mg and preferably about 100-300 $\mu$g of the emulsified hybrid should be administered intramuscularly, subcutaneously or intravenously.

An alternate means for administering this lipophilic immunogen is as part of liposome or micelle. Any of a number of liposme forming lipids known in the art may be utilized for this purpose with the effect of carrying the hybrid of this invention into the biological system.

The following examples are provided to further illustrate the invention and should not be construed as limiting thereon.

EXAMPLE 1

Preparation of cPND571:

A. Preparation of the Linear 28mer:

The 28mer :

```
 But But       Trt But      Boc Mtr Boc Mtr       Boc
 |   |         |   |        |   |   |   |         |
H-Ser Ser Nle Cys Tyr Asn Lys Arg Lys Arg Ile His -

                 Mtr          But But But Boc
                 |            |   |   |   |
Ile Gly Pro Gly Arg Ala Phe Tyr Thr Thr Lys Asn -

             Trt
             |
Ile Ile Gly Cys - OPKA resin      [Seq. Id:18:]
```

was assembled on the MILLIGEN #9050 synthesizer, starting with Fmoc-L-Cys(Trt)-OPKA resin (2.273 g, 0.2mmole, MILLIGEN batch 100234, 0.088 meq./g).

The resin was dry packed in a 1 x 10 cm column. Reagents were Fmoc-L-Pfp esters (except for Ser and Thr which were dHBT esters), and were used in four fold excess in NMP solution. Side chain protection was as shown above. Double couplings were provided at $Lys^{9}$, $Ile^{11}$, $Ile^{13}$, $Gly^{14}$, $Pro^{15}$, $Gly^{16}$, $Arg^{17}$, $Phe^{19}$, $Lys^{23}$, $Ile^{25}$, and $Ile^{26}$.

The free terminal amino derivatized resin was obtained as a pale tan powder (2.815g) after methylene chloride and ether washing and drying. An aliquot of the derivatized resin (140 mg, about 0.01 meq.) was suspended in 95% TFA/4% ethane dithiol/1% thioanisole (2.0 mL) at 24 °C and cleavage allowed to occur over 16 hours. The mixture was then filtered and the insolubles were washed with 100% TFA (2x2mL) and the combined filtrates were evaporated. The residual oily gum was triturated with ether (3x2mL) to give the crude 28mer as an insoluble tan powder (26.9 mg).

Analytical HPLC on a 0.46 x 25 cm VYDAC $C_{18}$ reverse phase column eluted with 22% acetonitrile in aq. 0.1% TFA, of 4 µl of a 50 µg sample dissolved in 50 µL of aq. 0.1% TFA/20% acetonitrile, monitored at 215 and 260 nm, A = 0.05, 2.0 mL/min. gave a major product peak at 17.28 minutes. After allowing the uninjected aliquot to sit at room temperature for 2 days, an aliquot was analyzed as above, and the major peak was at 8.14 minutes, demonstrating that the linear peptide was oxidizing to the disulfide.

B. Acylation of the Linear 28mer with $Pam_3$-OH:

$(Pam)_3$Cys-OH (173 mg, 0.19 mmole) was dissolved in NMP (1.0 mL) at 24 °C slowly to give a clear, pale brown solution upon sonication and stirring. 4-Methyl morpholine (0.285 mmole) was added, followed by solid BOP (84 mg, 0.19 mmole) and HOBt (25.7 mg, 0.19 mmole). The mixture was again sonicated and stirred (5 min. 24 °C) to obtain a clear solution. To the viscous solution was added more NMP (5 mL) and then mixed with the dry derivatized resin prepared above (1.337g, about 0.095 meq).

The thick reaction slurry was stirred at 24 °C for 17 hours. A few resin particles were removed, washed sequentially with NMP, methylene chloride, and then air dried. Analysis by Kaiser test (ninhydrin reagent, Kaiser et al., Anal. Biochem. 34, 595-598 (1970)) gave a blue color indicating the presence of residual free amino groups. The acylation reaction was allowed to proceed for another 18 hours, at which point a solution of BOP (42 mg, 0.095 mmole), HOBt (12.8 mg, 0.095 mmole) and 4-methyl morpholine (0.143 mmole) was added in NMP (1 mL), and the reaction allowed to proceed further. At t = 41 hours, the Kaiser test was

negative. The reaction was filtered and the resin was thoroughly washed with NMP (4 x 10 mL), methylene chloride (3 x 10 mL) and ether (3 x 10 mL). The resin was then dried to yield a pale tan powder (1.369 g).

C. Cleavage from the Resin and Product Recovery:

The derivatized, acylated resin was suspended in 95% TFA/4% ethanedithiol/1% thioanisole (15 mL) at 24 °C, forming a bright yellow solution. Cleavage was allowed to proceed for 20 hours with mixing.

The yellow slurry was filtered and the insolubles extracted with 100% TFA (3x10 mL). The combined filtrates were evaporated leaving a colorless, oily gum. This material was triturated with ether (4x3 mL), and the insoluble product was recovered by filtration. The product was dried to give a pale tan powder (265 mg).

A 0.46 x 25 cm VYDAC $C_4$ reverse phase column, heated electrically to 50 °C was equilibrated with aq. 0.1% TFA/63% acetonitrile. A 0.65 mg sample was dissolved by sonication in aq. 0.1% TFA/70% acetonitrile (0.1 mL), and injected. The injected sample was gradient eluted with aq. 0.1% TFA/63% - 70% acetonitrile over 30 minutes, and monitored at 215 nm, A = 3.00, 2.0 mL/min to isolate the product. A major peak eluting at 13.93 minutes was collected and concentrated by evaporation, followed by lyophilization. This same procedure was repeated with larger amounts of sample, and the material eluting at about 14 minutes was combined. Preparative HPLC on a 1.0 x 25 cm column gave similar results, and the combined product yield of cPND571 was 10.6 mg after lyophilization.

FAB-MS of a 0.4 mg sample gave $[M+H]^+$ = 4087, and $[M+Na]^+$ = 4109, which corresponds nicely with the calculated mass of 4088 for $C_{195}H_{329}N_{45}O_{43}S_3$, which is the mass of the cyclized material. Amino acid analysis revealed:

| amino acid | theoretical | found |
|---|---|---|
| Thr | 2 | 2.05 |
| Ser | 2 | 1.97 |
| Pro | 1 | 1.06 |
| Gly | 3 | 3.28 |
| Ala | 1 | 1.000 |
| 1/2Cystine | 2 | ----- |
| Ile | 4 | 3.78 |
| Tyr | 2 | 1.49 |
| Phe | 1 | 1.02 |
| His | 1 | 1.00 |
| Lys | 3 | 3.06 |
| Arg | 3 | 2.94 |
| Asn | 2 | 2.20 |
| Nle | 1 | 0.71 |

The mass of the product suggests that the deprotected, acylated material cyclized during heated chromatography. This was confirmed by analytic HPLC on a 0.25 x 1cm VYDAC $C_4$ column in aq. 0.1% TFA/63% acetonitrile, monitored at 215 nm, A = 0.05, 2.0 mL/min, to give a major peak at 13.49 minutes. Ellman assay showed the post-chromatography product obtained above had no free sulfydryls. Analytical HPLC as above of deprotected, acylated material which had not been chromatographed gave a major peak eluting at 15.23 minutes, and a strong positive result upon Ellman analysis.

EXAMPLE 2

Protocol for Inoculation of Animals with the $(Pam)_3$Cys-Ser-Ser-cPND Hybrid of this Invention:

The inoculum is prepared by making an emulsion in physiologic saline at a final concentration of about 300 µg/ml. Alternatively, a liposome incorporating the hybrid at about 300 µg/ml may be prepared.

African green monkeys or rabbits are individually inoculated with three 300 µg doses or three 100 µg doses of the hybrid. The doses are delivered one month apart (week 0, 4 and 8). The animals are bled at intervals of two weeks. Serum samples are prepared from each bleed to assay for the development of specific antibodies as described in the subsequent examples.

EXAMPLE 3

Analysis of Sera for Anti-HIV cPND IgG Antibodies:

Each serum sample is analyzed by enzyme-linked immunoadsorbent assay (ELISA). Polystyrene microtiter plates are coated with 0.5 $\mu$g per well of the synthetic peptide in phosphate-buffered physiological saline (PBS) at 4°C. Each well is then washed with PBS containing 0.05% TWEEN-20 (PBS-T). Test serum, diluted serially in PBS-T, is added to the peptide-containing wells and allowed to react with the adsorbed peptide for one hour at 36°C. After washing with PBS-T, alkaline phosphatase-conjugated goat anti-human IgG is added to the test wells and allowed to react for one hour at 36°C. The wells are then washed extensively in PBS-T. Each well receives 0.1% p-nitrophenyl phosphate in 10% diethanolamine, pH 9.8, containing 0.5 mM $MgCl_2 \cdot 6H_2O$. The ensuing reaction is allowed to proceed at room temperature for 30 minutes, at which time it is terminated by the addition of 3.0 N NaOH.

The greater the interaction of antibodies in the test serum with the peptide substrate, the greater is the amount of alkaline phosphatase bound onto the well. The phosphatase enzyme mediates the breakdown of p-nitrophenyl phosphate into a molecular substance which absorbs light at a wavelength of 405 nm. Hence, there exists a direct relationship between the absorbance at 405 nm of light at the end of the ELISA reaction and the amount of peptide-bound antibody.

EXAMPLE 4

Analysis of Sera for Activity which Specifically Neutralizes HIV Infectivity:

Virus-neutralizing activity is determined with an assay described by Robertson et al., J. Virol. Methods 20: 195-202 (1988). The assay measures specific HIV-neutralizing activity in test serum. The assay is based on the observation that MT-4 cells, a human T-lymphoid cell line, are readily susceptible to infection with HIV and, after a period of virus replication, are killed as a result of the infection.

The test serum is treated at 56°C for 60 minutes prior to the assay. This treatment is required to eliminate non-specific inhibitors of HIV replication. Heat treated serum, serially diluted in RPMI-1640 cell culture medium, is mixed with a standard infection dose of HIV. The dose is determined prior to the assay as containing the smallest quantity of virus required to kill all the MT-4 cells in the assay culture after a period of 7-8 days. The serum-virus mixture is allowed to interact for one hour at 37°C. It then is added to $1.0 \times 10^5$ MT-4 cells suspended in RPMI-1640 growth medium supplemented with 10% fetal bovine serum. The cultures are incubated at 37°C in a 5% $CO_2$ atmosphere for 7 days.

At the end of the incubation period, a metabolic dye, DTT, is added to each culture. This dye is yellow in color upon visual inspection. In the presence of live cells, the dye is metabolically processed to a molecular species which yields a blue visual color. Neutralized HIV cannot replicate in the target MT-4 cells and therefore does not kill the cells. Hence, positive neutralization is assessed by the development of blue color following addition of the metabolic dye.

## Claims

1. A hybrid immunogen comprising a cyclic HIV principal neutralizing determinant, cPND, and a synthetic lipopeptide analog of the E. coli or S. willmorei lipopeptide.

2. The hybrid immunogen of Claim 1 wherein the synthetic lipopeptide analog is triacyl-cysteinyl-seryl-serine, $(PAM)_3$Cys Ser Ser.

3. The hybrid immunogen of Claim 2 wherein the triacyl Cys Ser Ser is linked to an HIV cPND comprising the sequence Gly Pro Gly Arg. [Seq. ID: 1:].

**4.** The hybrid immunogen of Claim 3 having the formula [Seq. Id:19:]:

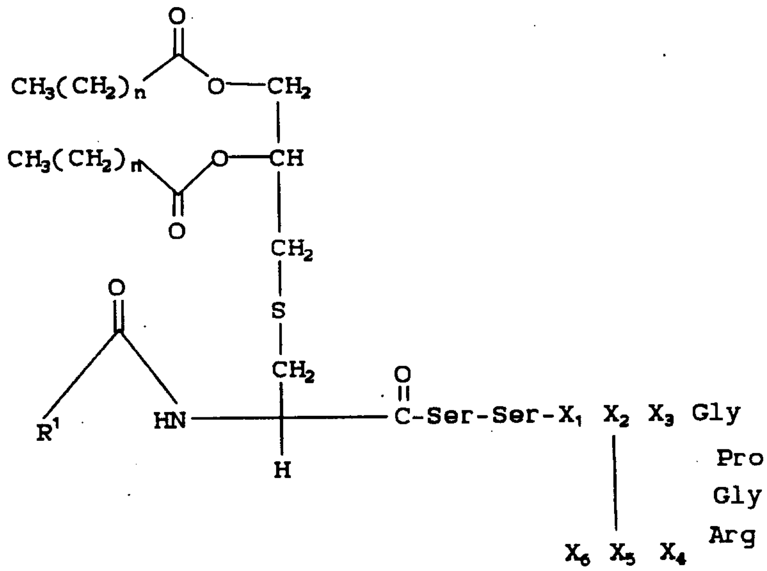

wherein:

- $X_1$ is a bond, an amino acid, or a peptide of 2-5 amino acids;
- $X_2$ is an amino acid bonded to $X_5$;
- $X_3$ is 1 to 25 amino acids long, selected from the sequence of amino acids found in any of the common isolates of HIV gp120, amino terminal and adjacent to the Gly Pro Gly Arg sequence;
- $X_4$ is a bond or 1 to 25 amino acids long, selected from the sequence of amino acids of any of the common isolates of HIV gp120, carboxy-terminal and adjacent to the Gly Pro Gly Arg sequence;
- $X_5$ is an amino acid bonded to $X_2$;
- $X_5$ is hydroxyl,<br>$-NH_2$, or<br>between 1 and 10 amino acids long;
- n is 1-20; and
- $R^1$ is $CH_3(CH_2)_n$- or $Cl_3CCH_2O$-.

**5.** The hybrid immunogen of Claim 4 having the formula [Seq. Id:20:]:

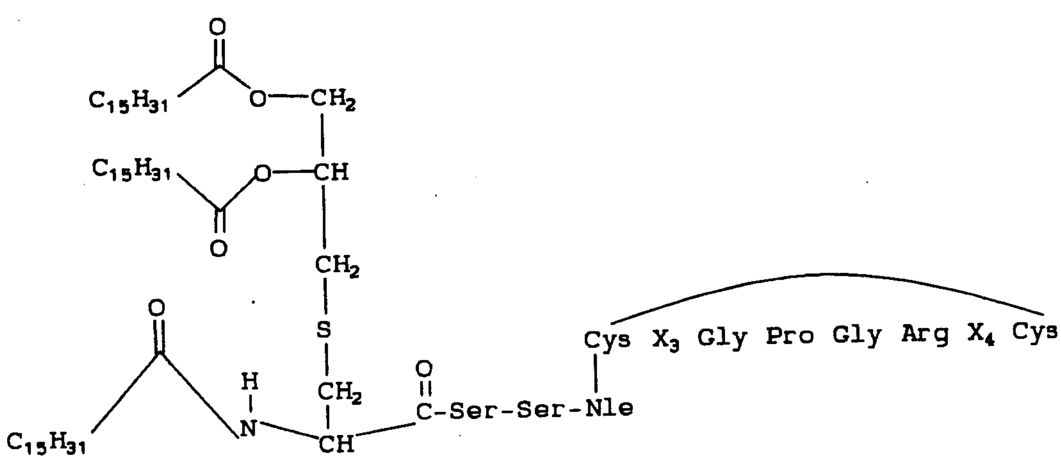

wherein $X_3$ and $X_4$ are as defined in Claim 4.

**6.** The hybrid immunogen of Claim 5 wherein:
$X_3$ is selected from:
Ile, His Ile, Ile His Ile, Arg Ile His Ile [Seq. Id:2:],
Lys Arg Ile His Ile [Seq. Id:3:], Arg Lys Arg Ile His Ile [Seq. Id:4:],
Lys Arg Lys Arg Ile His Ile [Seq. Id:5:], Asn Lys Arg Lys Arg Ile His Ile [Seq. Id:6:], Tyr Asn Lys Arg Lys Arg Ile His Ile [Seq. Id:7:],
Arg, Gln Arg, and Thr Arg Pro Asn Asn Asn Thr- Arg Lys Ser Ile His Ile [Seq. Id:15:]; $X_4$ selected from:
Ala,
Ala Phe, Ala Phe Tyr, Ala Phe Tyr Thr [Seq. Id:8:],
Ala Phe Tyr Thr Thr [Seq. Id:9:], Ala Phe Tyr Thr Thr Lys [Seq. Id:10:],
Ala Phe Tyr Thr Thr Lys Asn [Seq. Id:11:], Ala Phe Tyr Thr Thr-Lys Asn Ile [Seq. Id:12:], Ala Phe Tyr Thr Thr Lys Asn Ile Ile [Seq. Id:13:],
Ala Phe Tyr Thr Thr Lys Asn Ile Ile Gly [Seq. Id:14:], and Tyr Thr Thr Gly Glu Ile Gly Asp Ile Arg-Gln Ala His [Seq. Id:16:]; and the stereochemistry about the 2-palmitoyl residue in 2R.

**7.** The hybrid immuogen of Claim 1 having the formula, [Seq.ID:17:]:

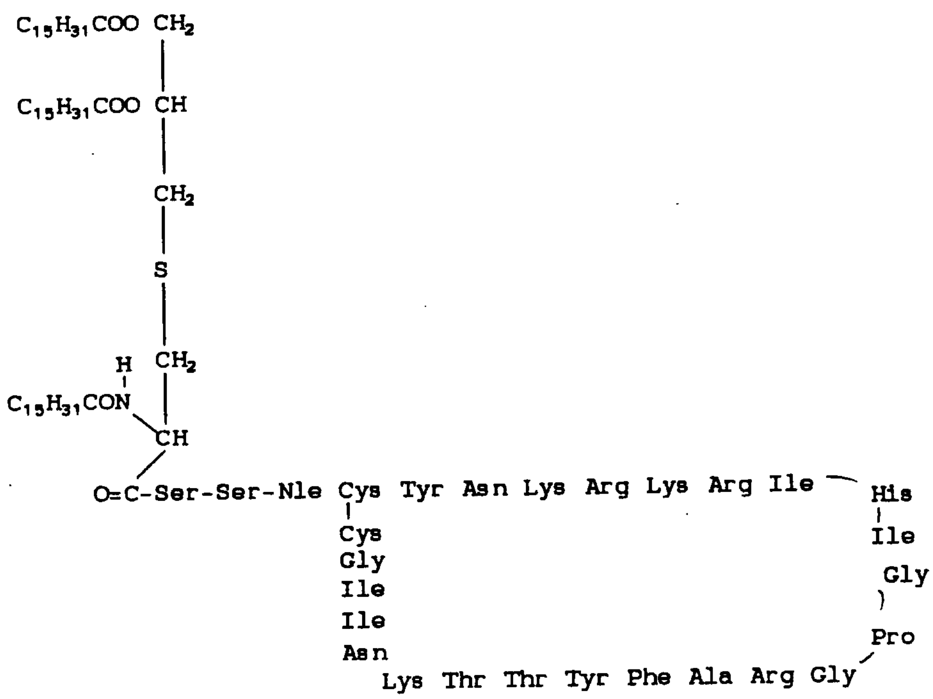

**8.** The immunogen of Claim 7 wherein the compound is the pure 2R enantiomeric form.

**9.** A pharmaceutical composition comprising a hybrid immunogen as claimed in Claim 1 and a physiologically acceptable carrier.

**10.** The composition of Claim 9 wherein the carrier is a liposome.

**11.** The use of a hybrid immunogen as claimed in Claim 1 for the manufacture of a medicament for inducing immune responses.

SEQUENCE LISTING

(2) INFORMATION FOR SEQ ID NO:1:

(i) SEQUENCE CHARACTERISTICS:

(A) LENGTH: 4 amino acids

(B) TYPE: amino acid

(D) TOPOLOGY: both

(ii) MOLECULE TYPE: peptide

(xi) SEQUENCE DESCRIPTION: SEQ ID NO:1:

Gly Pro Gly Arg

1

(2) INFORMATION FOR SEQ ID NO:2:

(i) SEQUENCE CHARACTERISTICS:

(A) LENGTH: 4 amino acids

(B) TYPE: amino acid

(D) TOPOLOGY: both

(ii) MOLECULE TYPE: peptide

(xi) SEQUENCE DESCRIPTION: SEQ ID NO:2:

```
Arg Ile His Ile
1
```

(2) INFORMATION FOR SEQ ID NO:3:

(i) SEQUENCE CHARACTERISTICS:

(A) LENGTH: 5 amino acids

(B) TYPE: amino acid

(D) TOPOLOGY: both

(ii) MOLECULE TYPE: peptide

(xi) SEQUENCE DESCRIPTION: SEQ ID NO:3:

```
Lys Arg Ile His Ile
1               5
```

(2) INFORMATION FOR SEQ ID NO:4:

(i) SEQUENCE CHARACTERISTICS:

(A) LENGTH: 6 amino acids

(B) TYPE: amino acid

(D) TOPOLOGY: both

(ii) MOLECULE TYPE: peptide

(xi) SEQUENCE DESCRIPTION: SEQ ID NO:4:

```
Arg Lys Arg Ile His Ile
1               5
```

(2) INFORMATION FOR SEQ ID NO:5:

(i) SEQUENCE CHARACTERISTICS:

(A) LENGTH: 7 amino acids

(B) TYPE: amino acid

(D) TOPOLOGY: both

(ii) MOLECULE TYPE: peptide

(xi) SEQUENCE DESCRIPTION: SEQ ID NO:5:

```
Lys Arg Lys Arg Ile His Ile
1               5
```

(2) INFORMATION FOR SEQ ID NO:6:

(i) SEQUENCE CHARACTERISTICS:

(A) LENGTH: 8 amino acids

(B) TYPE: amino acid

(D) TOPOLOGY: both

(ii) MOLECULE TYPE: peptide

(xi) SEQUENCE DESCRIPTION: SEQ ID NO:6:

Asn Lys Arg Lys Arg Ile His Ile
1 5

(2) INFORMATION FOR SEQ ID NO:7:

(i) SEQUENCE CHARACTERISTICS:

(A) LENGTH: 9 amino acids

(B) TYPE: amino acid

(D) TOPOLOGY: both

(ii) MOLECULE TYPE: peptide

(xi) SEQUENCE DESCRIPTION: SEQ ID NO:7:

Tyr Asn Lys Arg Lys Arg Ile His Ile
1 5

(2) INFORMATION FOR SEQ ID NO:8:

(i) SEQUENCE CHARACTERISTICS:

(A) LENGTH: 4 amino acids

(B) TYPE: amino acid

(D) TOPOLOGY: both

(ii) MOLECULE TYPE: peptide

(xi) SEQUENCE DESCRIPTION: SEQ ID NO:8:

Ala Phe Tyr Thr
1

(2) INFORMATION FOR SEQ ID NO:9:

(i) SEQUENCE CHARACTERISTICS:

(A) LENGTH: 5 amino acids

(B) TYPE: amino acid

(D) TOPOLOGY: both

(ii) MOLECULE TYPE: peptide

(xi) SEQUENCE DESCRIPTION: SEQ ID NO:9:

Ala Phe Tyr Thr Thr
1 5

(2) INFORMATION FOR SEQ ID NO:10:

(i) SEQUENCE CHARACTERISTICS:
(A) LENGTH: 6 amino acids
(B) TYPE: amino acid
(D) TOPOLOGY: both

(ii) MOLECULE TYPE: peptide

(xi) SEQUENCE DESCRIPTION: SEQ ID NO:10:

Ala Phe Tyr Thr Thr Lys
1 5

(2) INFORMATION FOR SEQ ID NO:11:

(i) SEQUENCE CHARACTERISTICS:
(A) LENGTH: 7 amino acids
(B) TYPE: amino acid
(D) TOPOLOGY: both

(ii) MOLECULE TYPE: peptide

(xi) SEQUENCE DESCRIPTION: SEQ ID NO:11:

Ala Phe Tyr Thr Thr Lys Asn
1 5

(2) INFORMATION FOR SEQ ID NO:12:

(i) SEQUENCE CHARACTERISTICS:
(A) LENGTH: 8 amino acids
(B) TYPE: amino acid
(D) TOPOLOGY: both

(ii) MOLECULE TYPE: peptide

(xi) SEQUENCE DESCRIPTION: SEQ ID NO:12:

Ala Phe Tyr Thr Thr Lys Asn Ile
1 5

(2) INFORMATION FOR SEQ ID NO:13:

(i) SEQUENCE CHARACTERISTICS:
(A) LENGTH: 9 amino acids
(B) TYPE: amino acid
(D) TOPOLOGY: both

(ii) MOLECULE TYPE: peptide

(xi) SEQUENCE DESCRIPTION: SEQ ID NO:13:

Ala Phe Tyr Thr Thr Lys Asn Ile Ile
1 5

(2) INFORMATION FOR SEQ ID NO:14:

(i) SEQUENCE CHARACTERISTICS:
(A) LENGTH: 10 amino acids
(B) TYPE: amino acid
(D) TOPOLOGY: both

(ii) MOLECULE TYPE: peptide

(xi) SEQUENCE DESCRIPTION: SEQ ID NO:14:

Ala Phe Tyr Thr Thr Lys Asn Ile Ile Gly
1 5 10

(2) INFORMATION FOR SEQ ID NO:15:

(i) SEQUENCE CHARACTERISTICS:
(A) LENGTH: 13 amino acids
(B) TYPE: amino acid
(D) TOPOLOGY: both

(ii) MOLECULE TYPE: peptide

(xi) SEQUENCE DESCRIPTION: SEQ ID NO:15:

Thr Arg Pro Asn Asn Asn Thr Arg Lys Ser Ile His Ile
1 5 10

(2) INFORMATION FOR SEQ ID NO:16:

(i) SEQUENCE CHARACTERISTICS:
(A) LENGTH: 14 amino acids
(B) TYPE: amino acid
(D) TOPOLOGY: both

(ii) MOLECULE TYPE: peptide

(xi) SEQUENCE DESCRIPTION: SEQ ID NO:16:

```
Tyr Thr Thr Gly Glu Ile Ile Gly Asp Ile Arg Gln Ala His
1               5                   10
```

(2) INFORMATION FOR SEQ ID NO:17:

(i) SEQUENCE CHARACTERISTICS:
(A) LENGTH: 29 amino acids
(B) TYPE: amino acid
(D) TOPOLOGY: both

(ii) MOLECULE TYPE: peptide

(ix) FEATURE:
(A) NAME/KEY: Disulfide-bond
(B) LOCATION: 5..29

(ix) FEATURE:
(A) NAME/KEY: Modified-site
(B) LOCATION: 4
(D) OTHER INFORMATION: /label= NORLEUCINE

(ix) FEATURE:
(A) NAME/KEY: Modified-site
(B) LOCATION: 1
(D) OTHER INFORMATION: /label= ACYLATION
/note= "THE CYSTEINE AT THIS SITE IS ATTACHED TO A DIACYLGLYCEROL THROUGH A THIOLESTER LINKAGE, AND IS ALSO ACYLATED AT THE FREE CYSTEINE AMINO GROUP

(ix) FEATURE:
(A) NAME/KEY: Thiolester-bond
(B) LOCATION: 1
(D) OTHER INFORMATION: /label= THIOLESTER
/note= "THE CYSTEINE IS LINKED TO A DIACYLGLYCEROL MOIETY THROUGH A THIOLESTER INCORPORATING THE SULFUR SIDE-CHAIN OF CYSTEINE"

(xi) SEQUENCE DESCRIPTION: SEQ ID NO:17:

Cys Ser Ser Leu Cys Tyr Asn Lys Arg Lys Arg Ile His Ile Gly Pro Gly
1 5 10 15

Arg Ala Phe Tyr Thr Thr Lys Asn Ile Ile Gly Cys
20 25

(2) INFORMATION FOR SEQ ID NO:18:

(i) SEQUENCE CHARACTERISTICS:
(A) LENGTH: 28 amino acids
(B) TYPE: amino acid
(D) TOPOLOGY: both

(ii) MOLECULE TYPE: peptide

(ix) FEATURE:
(A) NAME/KEY: Modified-site
(B) LOCATION: 3
(D) OTHER INFORMATION: /label= NORLEUCINE

(xi) SEQUENCE DESCRIPTION: SEQ ID NO:18:

Ser Ser Leu Cys Tyr Asn Lys Arg Lys Arg Ile His Ile Gly Pro Gly
1 5 10 15

Arg Ala Phe Tyr Thr Thr Lys Asn Ile Ile Gly Cys
20 25

(2) INFORMATION FOR SEQ ID NO:19:

(i) SEQUENCE CHARACTERISTICS:
(A) LENGTH: 13 amino acids
(B) TYPE: amino acid
(D) TOPOLOGY: circular

(ii) MOLECULE TYPE: peptide

(ix) FEATURE:
(A) NAME/KEY: Modified-site
(B) LOCATION: 1
(D) OTHER INFORMATION: /label= ACYLATED
/note= "ACYLATION SITE"

(ix) FEATURE:
(A) NAME/KEY: Peptide
(B) LOCATION: 4
(D) OTHER INFORMATION: /label= X1
/note= "A BOND, AMINO ACID, OR PEPTIDE OF 2-5 AMINO ACIDS"

(ix) FEATURE:
(A) NAME/KEY: Modified-site
(B) LOCATION: 5
(D) OTHER INFORMATION: /label= X2
/note= "AMINO ACID BONDED TO X5"

(ix) FEATURE:
(A) NAME/KEY: Peptide
(B) LOCATION: 6
(D) OTHER INFORMATION: /label= X3
/note= "AMINO ACID OR PEPTIDE UP TO 25 AMINO ACIDS LONG"

(ix) FEATURE:
(A) NAME/KEY: Peptide
(B) LOCATION: 11
(D) OTHER INFORMATION: /label= X4
/note= "A BOND, AN AMINO ACID, OR A PEPTIDE UP TO 25 AMINO ACIDS LONG"

(ix) FEATURE:
(A) NAME/KEY: Modified-site
(B) LOCATION: 12
(D) OTHER INFORMATION: /label= X5
/note= "AN AMINO ACID BONDED TO X2"

(ix) FEATURE:
(A) NAME/KEY: Peptide
(B) LOCATION: 13
(D) OTHER INFORMATION: /label= X6
/note= "-OH, -NH2, AN AMINO ACID, OR A PEPTIDE OF 2-10 AMINO ACIDS"

(xi) SEQUENCE DESCRIPTION: SEQ ID NO:19:

```
Cys Ser Ser Xaa Xaa Xaa Gly Pro Gly Arg Xaa Xaa Xaa
1               5                   10
```

```
(2) INFORMATION FOR SEQ ID NO:20:

    (i) SEQUENCE CHARACTERISTICS:
        (A) LENGTH: 12 amino acids
        (B) TYPE: amino acid
        (D) TOPOLOGY: circular

    (ii) MOLECULE TYPE: peptide

    (ix) FEATURE:
        (A) NAME/KEY: Modified-site
        (B) LOCATION: 1
        (D) OTHER INFORMATION: /label= ACYLATION
              /note= "THE CYSTEINE IS ACYLATED"

    (ix) FEATURE:
        (A) NAME/KEY: Modified-site
        (B) LOCATION: 3
        (D) OTHER INFORMATION: /label= NORLEUCINE

    (ix) FEATURE:
        (A) NAME/KEY: Disulfide-bond
        (B) LOCATION: 5..12

    (ix) FEATURE:
        (A) NAME/KEY: Peptide
        (B) LOCATION: 6
        (D) OTHER INFORMATION: /label= X3
              /note= "AN AMINO ACID OR A PEPTIDE UP TO 25 AMINO
              ACIDS LONG"

    (ix) FEATURE:
        (A) NAME/KEY: Peptide
        (B) LOCATION: 11
        (D) OTHER INFORMATION: /label= X4
              /note= "A BOND, AN AMINO ACID, OR A PEPTIDE OF UP
              TO 25 AMINO ACIDS"

    (xi) SEQUENCE DESCRIPTION: SEQ ID NO:20:

    Cys Ser Ser Leu Cys Xaa Gly Pro Gly Arg Xaa Cys
    1               5                   10
```